# EUROPEAN PATENT APPLICATION

(11) **EP 4 427 707 A1**
(43) Date of publication of application: **11.09.2024**
(21) Application number: 22889594.2
(22) Date of filing: 01.02.2022
(51) Int. Cl.: A61F 2/38

(54) **KNEE-JOINT SUPPORTING DEVICE**

(30) Priority: 02.11.2021 JP 2021179779
(71) Applicant: University of Miyazaki, Miyazaki-shi Miyazaki 889-2192 (JP)
(72) Inventor: YAMAKO Go, Miyazaki-shi, Miyazaki 889-2192 (JP); CHOSA Etsuo, Miyazaki-shi, Miyazaki 889-1692 (JP)
(74) Representative: Plougmann Vingtoft a/s
(86) International application number: PCT/JP2022/003718
(87) International publication number: WO 2023/079768

(57) **Abstract**

Provided is a supportive device for a knee joint which is less likely to be displaced. A artificial meniscus 10 is a supportive device for a knee joint which is circular. The artificial meniscus 10 is placed between a femur and a tibia. The artificial meniscus 10 includes a polycarbonate urethane. The artificial meniscus 10 comprises a shell 12 having flexibility and a core 11 having a rigidity higher than that of the shell 12. The artificial meniscus 10 is sewn to an articular capsule. A surface 12a of the artificial meniscus 10 is coated by MPC. Micro dimples 10d are formed on a surface of the artificial meniscus 10.

## Description

### Technical Field

The present invention relates to a supportive device for a knee joint.

### Background Art

Menisci are dynamic components having a role as cushions, e.g. for stabilization and load distribution of a knee joint. If a meniscus is injured by aging degeneration or doing sport, natural healing is difficult except for marginal portions having blood circulation, and it advances to osteoarthrosis (OA) by increasing load stress to cartilages. That is, meniscal injury is a significant disorder affecting a sporting life and it is important to prevent crisis of the OA for "extension of healthy life expectancy". However, if a meniscus is heavily injured, excision is the sole effective treatment, so a supportive device that can support the cartilages (an artificial meniscus for example) is required in field clinics in order to prevent crisis of the OA.

An artificial meniscus of anatomical type described in Non-Patent Literature 1 is known as a supportive device for a knee joint. Also, it is known that the artificial meniscus of the anatomical type does not function properly without accurate control of the installed position as described in Non-Patent Literature 2.

### Citation List

### Non Patent Literature

Non Patent Literature 1: A. C. T. Vrancken, W. Madej, G. Hannink, N. Verdonschot, T. G. van Tienen, P. Buma, "Short Term Evaluation of an Anatomically Shaped Polycarbonate Urethane Total Meniscus Replacement in a Goat Model", [retrieved on August 30, 2021], the Internet <URL:https://journals.plos.org/plosone/article?id=10.1371/journal.pone.0133138 >
Non Patent Literature 2: Duraisamy Shriram, Go Yamako, Gideon Praveen Kumar, Etsuo Chosa, Fangsen Cui, Karupppasamy Subburaj, "Non-anatomical placement adversely affects the functional performance of the meniscal implant: a finite element study", [retrieved on August 30, 2021], the Internet <URL:https://doi.org/10.1007/s10237-021-01440-w>

### Summary of Invention

### Technical Problem

However, conventional techniques have a problem that the supportive device is displaced.

For example, in the example of Non-Patent Literature 1, there is a problem that the artificial meniscus cannot hold the compressive load exerted on the artificial meniscus and is pushed out of the inserted position and displaced. A cause of the displacement is that an appropriate execution of fixing operation of the supportive device (for example, a technique to sew the artificial meniscus into an accurate position of the joint) is difficult.

The present invention is made in order to solve such a problem, and an object of the present invention is to provide a supportive device for a knee joint that is less likely to be displaced.

### Solution to Problem

An example of a supportive device for a knee joint related to the present invention is an annular supportive device.

The supportive device may be placed between a femur and a tibia.

The supportive device may be an artificial meniscus.

The supportive device may be circular.

The supportive device may include a polycarbonate urethane.

The supportive device may comprise: a shell having flexibility; and a core having a rigidity higher than that of the shell.

The supportive device may be sewn to an articular capsule.

A surface of the supportive device may be coated by MPC polymer (2-methacryloyloxyethyl phosphorylcholine).

A plurality of recesses and protrusions may be formed on a surface of the supportive device.

The present specification includes the content disclosed in Japanese Patent Application No. 2021-179779, of which the present application claims priority.

### Advantageous Effects of Invention

The supportive device for the knee joint in accordance with the present invention is less likely to be displaced.

### Brief Description of Drawings

Fig. 1 is a schematic structural diagram of a healthy knee joint (top view).
Fig. 2 is a schematic structural diagram of a healthy knee joint (side view).
Fig. 3 is a top view of an artificial meniscus related to a first embodiment of the present invention.
Fig. 4 is a cross-sectional view of the artificial meniscus of Fig. 3 taken along the IV-IV line.
Fig. 5 is a top view indicating a state wherein the artificial meniscus of Fig. 3 is placed within a knee joint.
Fig. 6 is side view indicating a state wherein the artificial meniscus of Fig. 3 is placed within a knee joint.
Fig. 7 is a schematic diagram showing a state wherein a meniscus has been excised in a knee joint.
Fig. 8 is a diagram explaining a function of the artificial meniscus of Fig. 3.
Fig. 9 is a diagram explaining a function of the artificial meniscus 10 in a construction different from Fig. 8.
Fig. 10 is a diagram showing an example of a cross-sectional shape of the artificial meniscus.
Fig. 11 is a diagram showing an example of a cross-sectional shape of the artificial meniscus.
Fig. 12 is a diagram showing an example of a cross-sectional shape of the artificial meniscus.
Fig. 13 is a diagram showing an example of a cross-sectional shape of the artificial meniscus.
Fig. 14 is a diagram showing a modified example of mounting manner of the artificial meniscus.
Fig. 15 is a diagram explaining about an experiment using an artificial meniscus related to an example of the present invention.

### Description of Embodiments

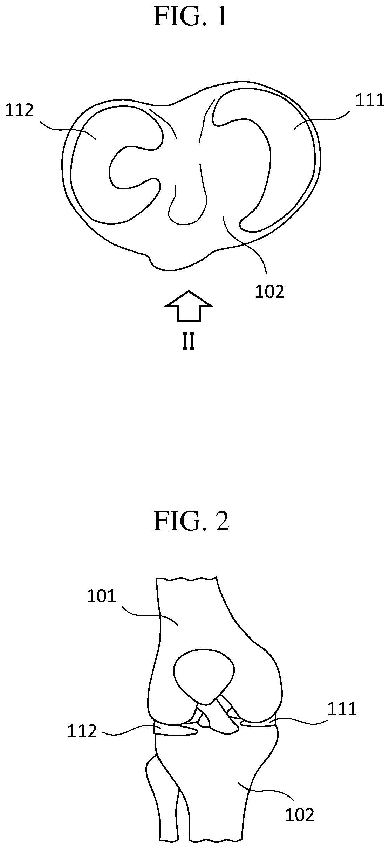

Embodiments of the present invention will be explained below based on the attached drawings. Figs. 1 and 2 are schematic structural diagrams of a healthy knee joint. Fig. 1 is a top view and a downward direction in the sheet corresponds to a forward direction of a human body. Fig. 2 is a side view seen from II direction in Fig. 2 (i.e. from a forward direction of the human body). A medial meniscus 111 and a lateral meniscus 112 are placed between a femur 101 and a tibia 102.

### [First Embodiment]

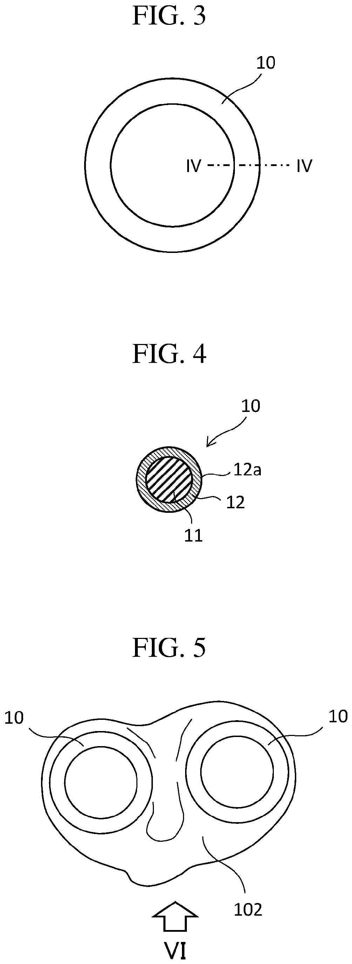

Fig. 3 is a top view of an artificial meniscus 10 related to a first embodiment of the present invention. The artificial meniscus 10 is a supportive device for a knee joint and is also referred to as a meniscal implant. The artificial meniscus 10 is formed to be annular. In particular, it is circular in the example of Fig. 3, i.e. in a shape symmetrical with respect to rotation of any angle about a predetermined axis.

Sizes of the artificial meniscus 10 can be designed in accordance with e.g. a structure of a knee joint of a patient to which it is to be applied, and for example, an inner diameter thereof can be within a range of 5 to 30 mm.

Fig. 4 is a cross-sectional view of the artificial meniscus 10 showing a cross section taken along the IV-IV line in Fig. 3. The artificial meniscus 10 comprises a core 11 and a shell 12, and the shell 12 is placed around the core 11. A cross-sectional diameter of the artificial meniscus 10 (for example, a diameter of an outer surface of the shell 12) can be within a range of 1 to 8 mm.

The shell 12 has flexibility and the core 11 has a rigidity higher than that of the shell 12. The core 11 is, for example, embedded inside the shell 12 by insert molding.

As a material of the shell 12, a biocompatible polymeric material can be used for example. As a biocompatible polymeric material, a polycarbonate urethane can be used for example. A Young's modulus of the shell 12 can be set to 11MPa or about 11MPa for example.

As a material of the core 11, a biocompatible polymeric material similar to the shell 12 can be used for example, and as a specific example, a polycarbonate urethane can be used. A Young's modulus of the core 11 can be 1.5 times or more of the Young's modulus of the shell 12. If the Young's modulus of the shell 12 is 11MPa as described above, the Young's modulus of the core 11 can be set to 17 MPa or about 17 MPa for example.

Thus, the artificial meniscus 10 can be produced with a polycarbonate urethane included therein for at least a portion. Flexibility of the artificial meniscus 10 can be realized appropriately by using the polycarbonate urethane so that stress can be relieved more appropriately.

A coating may be applied to a surface 12a of the shell 12 (i.e. a surface of the artificial meniscus 10). For example, the surface 12a may be coated by MPC (2-methacryloyloxyethyl phosphorylcholine). In this way, the surface 12a becomes smooth and lubricity is enhanced.

Also, a portion or an entire portion of the core 11 may be made of a radiopaque material. In this way, the position of the artificial meniscus 10 can be confirmed easily by roentgenography after the artificial meniscus 10 is installed within a knee joint.

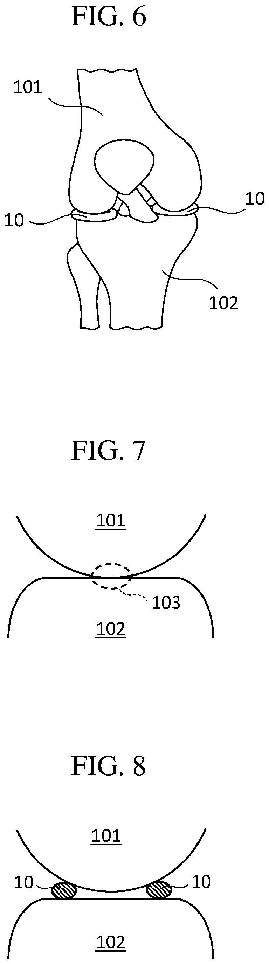

Figs. 5 and 6 show a state wherein the artificial meniscus 10 related to the first embodiment of the present invention is placed within the knee joint. Fig. 5 is a top view corresponding to Fig. 1 and a downward direction in the sheet corresponds to a forward direction of a human body. Fig. 6 is a side view corresponding to Fig. 2 and seen from VI direction in Fig. 5 (i.e. from a forward direction of the human body). A method of installation can be in accordance with a normal operation in a field of orthopedics.

The artificial meniscus 10 is placed within the knee joint as an implant, and in particular, placed between the femur 101 and the tibia 102. In the example of Figs. 5 and 6, the medial meniscus 111 and the lateral meniscus 112 in Figs. 1 and 2 are both completely excised and the two artificial menisci 10 are respectively placed corresponding to them. However, only one of the medial meniscus 111 and the lateral meniscus 112 may be replaced by the artificial meniscus 10.

Fig. 7 is a schematic diagram showing a state wherein the meniscus (the medial meniscus 111 or the lateral meniscus 112) has been excised from the knee joint. In this state, the femur 101 and the tibia 102 are in direct contact at a contact point 103 so that cartilages may be injured by attrition.

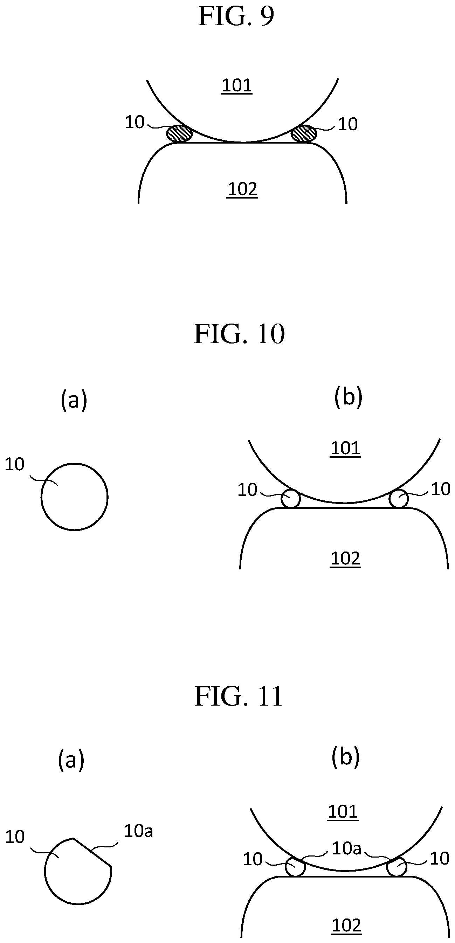

Fig. 8 is diagram explaining a function of the artificial meniscus 10. The artificial meniscus 10 reduces attrition of the cartilages by preventing direct contact between the femur 101 and the tibia 102 or by relieving the stress exerted between them. Upon this, the cross-sectional shape of the artificial meniscus 10 may be deformed due to the stress. Note that Fig. 8 represents this deformation by the cross-sectional shape being an ellipse. The same is applied to Fig. 9 which will be referred to later.

Also, the artificial meniscus 10 can be mounted easily by fitting it onto a condylar portion of the femur 101 because the artificial meniscus 10 is annular (see Fig. 3). Also, after being mounted, the artificial meniscus 10 is less likely to be displaced because the condylar portion of the femur 101 is fixed at the inner side of the artificial meniscus 10 (the position shown in Fig. 8) by vertical load. Thus, such problem as described in Non-Patent Literature 2 can be avoided.

Also, because of this, no fixing treatment such as suture is necessary, and in particular, deviation in mounting results depending on skills of operators can be suppressed. Further, if no fixing treatment such as suture is performed, an exchanging treatment can be performed easily in the cases e.g. where a size of the installed artificial meniscus 10 was not accurate. (Note that, it is also possible to perform a fixing treatment, as described later.)

Further, the artificial meniscus 10 is annular and can distribute the load in a circumferential direction, so it is robust with respect to hoop stress and less likely to deform. Accordingly, displacement due to excessive deformation is also less likely. A human meniscus has dynamical anisotropy by orienting collagen fibers richly in a circumferential direction so that it has a structure that is robust to the hoop stress and less likely to deform, and it can be said that the artificial meniscus 10 related to the present embodiment reproduces this feature well.

In the present embodiment, due to the shell-core structure shown in Fig. 4, the shell 12 distributes the load and the core 11 having a rigidity higher than that of the shell 12 can maintain stability of the structure.

In the present embodiment, the artificial meniscus 10 is annular, so it deforms in response to loads so that it is adapted to shapes of complicated curved surfaces in articular facets of knees different for respective patients. Accordingly, it can be adapted to various patients by a single type of shape.

Fig. 9 is a diagram explaining a function of the artificial meniscus 10 in a construction different from Fig. 8. Although the femur 101 and the tibia 102 are not in contact in the example of Fig. 8, the artificial meniscus 10 can be constructed with a greater inner diameter so that the femur 101 and the tibia 102 are in contact (more precisely, articular cartilages thereof are in contact with each other). Even in such a construction, the attrition in the cartilages is reduced because the load is reduced by the artificial meniscus 10.

The shape and sizes of the artificial meniscus 10 can be changed in various manners. Although it is circular in the example of Fig. 3, it does not have to be circular. For example, its curvature may change in accordance with a circumferential position of the loop, or it may contain a straight portion.

Also, although the shapes and the sizes of the two artificial menisci 10 are identical to each other in the example of Figs. 5 and 6, the shapes and/or sizes of the two artificial menisci 10 may differ from each other in the case wherein the two artificial menisci 10 are to be used within a single knee joint.

Figs. 10-12 show various examples of the cross-sectional shapes of the artificial meniscus 10. In these figures, (a) indicates a contour of the cross section of the artificial meniscus 10 and (b) indicates a state wherein the artificial meniscus 10 is placed between the femur 101 and the tibia 102 (in these figures, deformation in the cross section of the artificial meniscus 10 due to the stress is ignored).

In Fig. 10, the contour is circular and it corresponds to the cross-sectional shape of Fig. 4.

The contour of the cross section of the artificial meniscus 10 does not have to be circular. The curvature may change in accordance with a circumferential position of the contour. For example, the contour may include, as a portion, a straight portion 10a as in the modified example shown in Fig. 11. The straight portion 10a constitutes a partial conical surface within the artificial meniscus 10 at an inner side of the loop. As shown in Fig. 11(b), if it is placed so that the partial conical surface faces the condylar portion of the femur 101, the load exerted to a single point can be reduced by increasing the contact area between the artificial meniscus 10 and the femur 101.

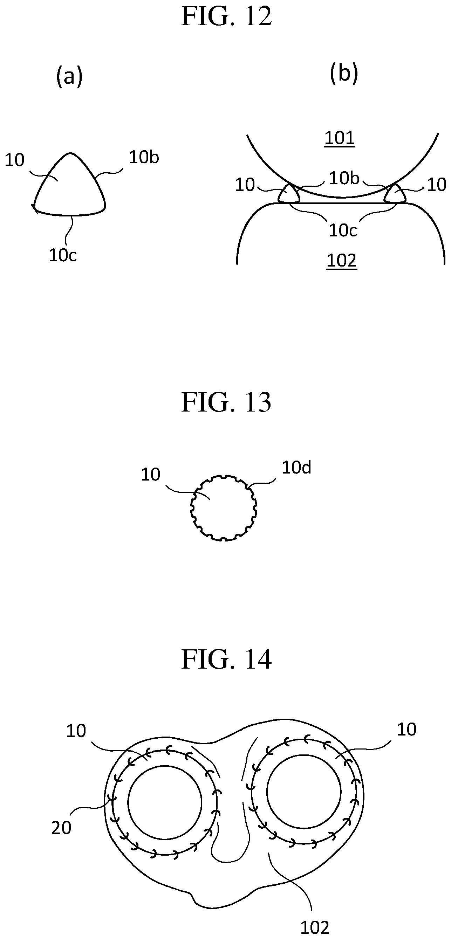

Also, as shown in Fig. 12 as a modified example, the contour may be a rounded polygonal shape (a rounded triangular shape in this example). The contour in Fig. 12 comprises a femur facing portion 10b facing the femur 101 and a tibia facing portion 10c facing the tibia 102. The femur facing portion 10b is a partial conical surface rounded in a loop and the tibia facing portion 10c is a circular surface rounded in a loop. As shown in Fig. 12, if the artificial meniscus 10 is placed so that the femur facing portion 10b is in contact with the femur 101 and the tibia facing portion 10c is in contact with the tibia 102, the load exerted to a single point can be reduced by increasing the contact area between the artificial meniscus 10 and the femur 101 and the tibia 102.

Fig. 13 is a diagram showing another example of a cross-sectional shape of the artificial meniscus 10. Micro dimples 10d, which are a plurality of recesses and protrusions, are formed on a surface of the artificial meniscus 10. Although the micro dimples 10d are formed to be many microscopic recessed portions in the example of Fig. 13, the shape is not limited thereto and may be many protruding portions (i.e. the shape does not have to correspond to the name "dimple"). Also, the recessed and protruding portions may coexist. By forming such recesses and protrusions, a liquid pool is formed between a surface of the artificial meniscus 10 and another structure (such as the femur 101, the tibia 102, etc.) so that lubricity is enhanced.

Although it is not shown, the cross-sectional shape of the artificial meniscus 10 may be different depending on a circumferential direction of the loop. For example, it may have a portion wherein the cross-sectional area is large and a portion wherein the cross-sectional area is small.

Fig. 14 shows a modified example of mounting manner of the artificial meniscus 10. In this example, the artificial meniscus 10 is sewn and fixed by using a surgical suture 20. For example, it is preferable to be sewn to an articular capsule. By performing such suture, prevention of displacement of the artificial meniscus 10 is further ensured. Note that the specific manner of the suture (suture position, number of sutures, etc.) is not limited to that shown in Fig. 14 and can be determined by the operator as needed.

Also, other manners can be used for fixation, although they are not shown. For example, a surgical suture and an anchor may be used or a screw may be used. For this purpose, the artificial meniscus may be provided with a through hole for attaching the anchor, the screw, etc.

### [Other Modifications]

Modifications below may be added to the artificial meniscus 10 (a supportive device for a knee joint) related to the first embodiment.

The artificial meniscus 10 may be of a construction that does not have a shell-core structure such as shown in Fig. 4. For example, an entire portion thereof may be constructed by a uniform material.

The supportive device for the knee joint related to the present invention is not limited to one used as replacement for a meniscus (i.e. an artificial meniscus). For example, it may be a supportive device used in combination, after a portion of a human meniscus is excised, with the remainder (meniscus supportive device). It may be a supportive device used in combination with a human meniscus without excising any portion of the human meniscus. Even in such cases, an existing meniscus can be supported by relieving stress and displacement can be prevented by having an annular construction shown in Fig. 3.

### [Experimental Example]

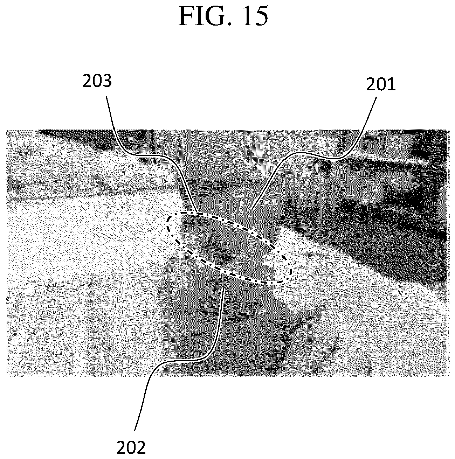

An example using the artificial meniscus related to an example of the present invention will be explained by using Fig. 15. In the experiment, a knee joint of a swine is used to install the artificial meniscus by excising a medial meniscus. In Fig. 15, the artificial meniscus (not appearing in the figure) is installed in a region 203 between the femur 201 and the tibia 202. It was easy to perform the installation.

The shape of the artificial meniscus used in the experiment is circular as exemplified in Figs. 3 and 4. Regarding the size, an inner diameter is 8.4 mm and a diameter of a cross section is 3 mm. A biocompatible polycarbonate urethane is used for the material.

In a state where the artificial meniscus is installed, the knee joint was moved manually and it was confirmed that the artificial meniscus did not fall off from the articular facet. Also, it was confirmed that there was no locking of the knee joint.

Thus, it was confirmed by this experiment that the stress around the cartilages can be relieved by using the artificial meniscus of appropriate size and material. It is therefore considered from the above that the artificial meniscus is effective in preventing crisis of the osteoarthrosis (OA).

### Industrial Applicability

The present invention can be utilized in treatment for a knee joint in a field of orthopedics, and in particular effective in treatment for meniscus injury.

### Reference Signs List

10 ARTIFICIAL MENISCUS (SUPPORTIVE DEVICE)
10A STRAIGHT PORTION
10B FEMUR FACING PORTION
10C TIBIA FACING PORTION
10D MICRO DIMPLE
11 CORE
12 SHELL
12A SURFACE
20 SURGICAL SUTURE
101 FEMUR
102 TIBIA
103 CONTACT POINT
111 MEDIAL MENISCUS
112 LATERAL MENISCUS

All publications, patents and patent applications referred to in the present specification are incorporated herein as they are by reference.

## Claims

1. A supportive device for a knee joint, wherein the supportive device is annular.

2. The supportive device according to claim 1, wherein the supportive device is placed between a femur and a tibia.

3. The supportive device according to claim 1 or 2, wherein the supportive device is an artificial meniscus.

4. The supportive device according to any of claims 1 to 3, wherein the supportive device is circular.

5. The supportive device according to any of claims 1 to 4, wherein the supportive device includes a polycarbonate urethane.

6. The supportive device according to any of claims 1 to 5, wherein the supportive device comprises:
a shell having flexibility; and
a core having a rigidity higher than that of the shell.

7. The supportive device according to any of claims 1 to 6, wherein the supportive device is sewn to an articular capsule.

8. The supportive device according to any of claims 1 to 7, wherein a surface of the supportive device is coated by MPC.

9. The supportive device according to any of claims 1 to 8, wherein a plurality of recesses and protrusions are formed on a surface of the supportive device.
